# EUROPEAN PATENT SPECIFICATION

(11) **EP 1 265 877 B1**
(45) Date of publication and mention of the grant of the patent: **12.11.2003**
(21) Application number: 01921284.4
(22) Date of filing: 23.02.2001
(51) Int. Cl.: C07D 301/12

(54) **METHOD FOR MAKING AN OXIRANE**
VERFAHREN ZUR HERSTELLUNG VON OXIRAN
PROCEDE DE FABRICATION D'UN OXIRANNE

(30) Priority: 10.03.2000 FR 0003207
(43) Date of publication of application: 18.12.2002
(73) Proprietor: SOLVAY (Société Anonyme), 1050 Bruxelles (BE)
(72) Inventor: SCHOEBRECHTS, Jean-Paul, B-1390 Grez-Doiceau (BE); STREBELLE, Michel, B-1150 Bruxelles (BE); CATINAT, Jean-Pierre, B-7131 Waudrez (BE)
(74) Representative: Vande Gucht, Anne
(86) International application number: EP0102139
(87) International publication number: WO01068623

(56) References cited:
- EP-A- 0 568 336
- EP-A- 0 700 887
- WO-A-99/48883
- FR-A- 2 070 406
- FR-A- 2 419 938
- PATENT ABSTRACTS OF JAPAN vol. 1995, no. 11, 26 décembre 1995 (1995-12-26) & JP 07 206835 A (IDEMITSU KOSAN CO LTD), 8 août 1995 (1995-08-08)

## Description

L'invention concerne un procédé de fabrication d'un oxiranne par réaction entre une oléfine et un composé peroxydé en présence d'un catalyseur et d'un diluant. Elle concerne plus particulièrement un procédé de fabrication de 1,2-époxypropane (oxyde de propylène) ou de 1,2-époxy-3-chloropropane (épichlorhydrine) par réaction entre le propylène ou le chlorure d'allyle et le peroxyde d'hydrogène.

Il est connu de fabriquer de l'oxyde de propylène par époxydation de propylène au moyen de peroxyde d'hydrogène dans un solvant et en présence d'un catalyseur de type TS-1, comme décrit par exemple dans la demande de brevet EP 0 568 336. Le méthanol est utilisé comme solvant dans les exemples.

Ce procédé connu présente l'inconvénient de conduire à la formation de sous-produits. En effet, lorsque l'oxyde de propylène est fabriqué, des sous-produits sont formés par réaction entre l'oxyde de propylène et l'eau ou le méthanol, et notamment le propylène glycol et des méthoxypropanols de formules CH₃-CHOH-CH₂-OCH₃ et CH₃-CH(OCH₃)-CH₂OH. Lorsque l'épichlorhydrine est fabriquée, des sous-produits sont formés par réaction entre l'épichlorhydrine et l'eau ou le méthanol, et notamment le 1-chloropropanediol et des chlorométhoxypropanols de formule ClCH₂-CHOH-CH₂-OCH₃ et Cl-CH₂-CH(OCH₃)-CH₂OH. La formation de sous-produits réduit la sélectivité du procédé et dès lors son rendement.

L'invention vise à éviter la formation de sous-produits et donc à fournir un procédé de sélectivité élevée, tout en gardant une activité (ou vitesse de réaction) élevée.

L'invention concerne dès lors un procédé de fabrication d'un oxiranne selon lequel on fait réagir, dans un diluant choisi parmi l'eau, les alcools et les cétones, une oléfine avec un composé peroxydé en présence d'un catalyseur à base de silicalite de titane de type TS-1 et en présence d'un nitrile.

Une des caractéristiques essentielles de l'invention réside dans la présence de nitrile dans le milieu d'époxydation. Il a en effet été constaté que la présence d'un nitrile, même en une faible teneur, permet de réduire fortement la formation de sous-produits tels que les méthoxypropanols. Par exemple, en ajoutant un nitrile dans le milieu d'époxydation, on peut réduire la quantité de sous-produits formés, par rapport à un procédé réalisé dans des conditions identiques mais en l'absence de nitrile, d'au moins 20 %, en particulier d'au moins 30 %, de préférence d'au moins 50 %. Dans certains cas, on peut réduire la quantité de sous-produits d'au moins 75 %. On peut ainsi atteindre une sélectivité en époxyde, exprimée par le rapport molaire entre l'époxyde formé sur la somme des sous-produits (exprimés en C3) plus l'époxyde, d'au moins 75 %, en particulier d'au moins 80 %, de préférence d'au moins 85 %, une sélectivité d'au moins 90 % étant particulièrement préférée.

La quantité de nitrile mise en oeuvre dans le procédé selon l'invention peut varier dans une large mesure. Des doses très faibles donnent déjà un effet significatif sur la formation de sous-produits. Des quantités trop élevées peuvent dans certains cas ne pas être souhaitées car elles résultent en une réduction de la vitesse de réaction. En général un bon compromis entre la vitesse et la sélectivité est obtenu avec un rapport molaire des quantités de nitrile et de diluant mises en oeuvre d'au moins 0,001 %. Ce rapport est en particulier d'au moins 0,005 %, de préférence d'au moins 0,01 %. Un rapport d'au moins 0,1 % donne les meilleurs résultats. Le rapport est habituellement d'au plus 50 %, en particulier d'au plus 45 %, plus particulièrement d'au plus 40 %. Ce rapport peut par exemple être inférieur à 34 %, en particulier d'au plus 30 %. Un rapport d'au plus 10 % est préféré. Un rapport d'au plus 5 % donne de bons résultats.

La quantité de nitrile mise en oeuvre dans le procédé selon l'invention est couramment telle que le rapport molaire nitrile/oléfine est d'au moins 0,00003, en particulier d'au moins 0,0003 et de préférence d'au moins 0,003. Ce rapport est habituellement d'au plus 1,35, en particulier d'au plus 0,9 et de préférence d'au plus 0,15.

Le nitrile mis en oeuvre dans le procédé selon l'invention peut être choisi parmi les nitriles aliphatiques saturés, linéaires ou branchés et parmi les nitriles aromatiques. On préfère les nitriles aliphatiques saturés. Généralement, le nitrile contient jusqu'à 10 atomes de carbone, de préférence de 2 à 7 atomes de carbone. On peut citer à titre d'exemples, l'acétonitrile et le pivalonitrile. L'acétonitrile est préféré. Les nitriles qui forment un azéotrope avec le diluant, tel que l'acétonitrile avec le méthanol, présentent l'avantage d'être faciles à recycler avec le diluant.

Le diluant mis en oeuvre dans le procédé selon l'invention est le plus souvent organique. Il peut être choisi parmi les alcools aliphatiques saturés, linéaires ou branchés. Le diluant alcoolique contient généralement jusqu'à 10 atomes de carbone, de préférence de 1 à 6 atomes de carbone. On peut citer à titre d'exemples le méthanol et l'éthanol. Le méthanol est préféré.

Le milieu d'époxydation dans lequel l'oléfine réagit avec le composé peroxydé en présence du catalyseur, du diluant alcoolique et du nitrile, contient le plus souvent également de l'eau. Le milieu d'époxydation comprend généralement une phase liquide, une phase gazeuse et le catalyseur à l'état solide. La phase liquide contient le diluant, le nitrile, l'oléfine dissoute, le composé peroxydé, une fraction de l'époxyde formé et de l'eau.

La quantité totale de diluant et de nitrile mise en oeuvre dans le procédé selon l'invention est généralement d'au moins 35 % en poids de la phase liquide définie ci-dessus, en particulier d'au moins 60 % en poids, par exemple d'au moins 75 % en poids. Cette quantité ne dépasse habituellement pas 99 % en poids, en particulier pas 95 % en poids.

Le rapport molaire entre les quantités d'oléfine et de composé peroxydé engagées dans le procédé selon l'invention est généralement d'au moins 0,1, en particulier d'au moins 1, et de préférence d'au moins 5. Ce rapport molaire est le plus souvent d'au plus 100, en particulier d'au plus 50 et de préférence d'au plus 25.

Le procédé selon l'invention peut être continu ou discontinu.

Dans le procédé selon l'invention, lorsqu'il est réalisé en continu, le composé peroxydé est généralement mis en oeuvre en une quantité d'au moins 0,005 mole par heure et par gramme de silicalite de titane, en particulier, d'au moins 0,01 mole par heure et par gramme de silicalite de titane. La quantité de composé peroxydé est habituellement inférieure ou égale à 2,5 moles par heure et par gramme de silicalite de titane et, en particulier, inférieure ou égale à 1 mole par heure et par gramme de silicalite de titane. Une préférence est montrée pour une quantité de composé peroxydé supérieure ou égale à 0,03 mole par heure et par gramme de silicalite de titane et inférieure ou égale à 0,25 mole par heure et par gramme de silicalite de titane.

Dans le procédé selon l'invention le composé peroxydé est avantageusement mis en oeuvre sous forme d'une solution aqueuse. En général, la solution aqueuse contient au moins 10 % en poids de composé peroxydé, en particulier au moins 20 % en poids. Elle contient le plus souvent au maximum 70 % en poids de composé peroxydé, en particulier 50 % en poids.

La température de la réaction entre l'oléfine et le composé peroxydé peut varier de 10 à 100 °C. Dans une variante avantageuse, elle est supérieure à 35 °C pour remédier à la désactivation progressive du catalyseur. La température peut être supérieure ou égale à 40 °C et de préférence supérieure ou égale à 45 °C. Une température supérieure ou égale à 50 °C est tout particulièrement préférée. La température de réaction est de préférence inférieure à 80 °C.

Dans le procédé selon l'invention, la réaction entre l'oléfine et le composé peroxydé peut avoir lieu à pression atmosphérique. Elle peut également se dérouler sous pression. Généralement, cette pression n'excède pas 40 bar. Une pression de 20 bar convient bien en pratique.

Les composés peroxydés qui peuvent être utilisés dans le procédé selon l'invention sont les composés peroxydés contenant une ou plusieurs fonctions peroxyde (-OOH) qui peuvent libérer de l'oxygène actif et capables d'effectuer une époxydation. Le peroxyde d'hydrogène et les composés peroxydés qui peuvent produire du peroxyde d'hydrogène dans les conditions de la réaction d'époxydation conviennent bien. Le peroxyde d'hydrogène est préféré.

Lorsqu'on utilise du peroxyde d'hydrogène, il peut être intéressant de mettre en oeuvre dans le procédé selon l'invention une solution aqueuse de peroxyde d'hydrogène à l'état brut, c'est-à-dire non épurée. Par exemple, on peut mettre en oeuvre une solution obtenue par simple extraction avec de l'eau substantiellement pure du mélange issu de l'oxydation d'au moins une alkylanthrahydroquinone (procédé appelé "procédé AO auto-oxydation") sans traitement ultérieur de lavage et/ou de purification. Ces solutions brutes de peroxyde d'hydrogène contiennent généralement de 0,001 à 10 g/l d'impuretés organiques exprimées en COT (Carbone Organique Total). Elles contiennent habituellement des cations métalliques (tels que des métaux alcalins ou alcalino-terreux, comme le sodium) et des anions (tels que les phosphates, nitrates) en des teneurs de 0,01 à 10 g/l.

L'oxiranne qui peut être préparé par le procédé selon l'invention est un composé organique comprenant un groupement répondant à la formule générale :

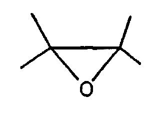

L'oxiranne contient généralement de 3 à 10 atomes de carbone, de préférence de 3 à 6 atomes de carbone. Les oxirannes qui peuvent être préparés de manière avantageuse par le procédé selon l'invention sont le 1,2-époxypropane et le 1,2-époxy-3-chloropropane.

Les oléfines qui conviennent bien dans le procédé selon l'invention contiennent généralement de 3 à 10 atomes de carbone et de manière préférée, 3 à 6 atomes de carbone. Elles sont de préférence non aromatiques. Le propylène, le butylène et le chlorure d'allyle conviennent bien. Le propylène et le chlorure d'allyle sont préférés.

Le silicalite de titane de type TS-1 utilisé dans le procédé selon l'invention est une zéolite au titane constituée d'oxydes de silicium et de titane et présentant une structure cristalline de type ZSM-5. Le silicalite de titane présente généralement une bande d'adsorption infrarouge à environ 950-960 cm⁻¹. Les silicalites de titane répondant à la formule xTiO₂(1-x)SiO₂ dans laquelle x est de 0,0001 à 0,5, de préférence de 0,001 à 0,05 donnent de bons résultats.

Dans le procédé selon l'invention, un gaz n'ayant pas d'influence négative sur la réaction d'époxydation peut également être ajouté au réacteur. En effet, dans la demande de brevet WO 99/48883 (dont le contenu est incorporé par référence dans la présente demande de brevet), la demanderesse a trouvé qu'en introduisant un composé gazeux dans le milieu réactionnel à un débit suffisant pour permettre d'entraîner l'oxiranne produit et de le sortir du réacteur en même temps que le composé gazeux, on diminue le temps de contact entre l'oxiranne produit et le milieu réactionnel d'époxydation. On évite ainsi également la formation de sous-produits et on augmente la sélectivité de l'époxydation.

Dans le procédé selon l'invention on peut utiliser tout type de réacteur, en particulier un réacteur de type boucle. Les réacteurs de type boucle à bullosiphon, dans lesquels la circulation du liquide et aussi éventuellement du catalyseur est obtenue par barbotage d'un gaz dans l'une des branches, conviennent bien. Ce type de réacteur est décrit dans la demande de brevet WO 99/48883 mentionnée ci-dessus.

Dans le procédé selon l'invention il peut s'avérer intéressant de contrôler le pH de la phase liquide. Par exemple, il peut être intéressant de maintenir le pH de la phase liquide lors de la réaction entre l'oléfine et le composé peroxydé à une valeur de 4,8 à 6,5, par exemple par addition d'une base (hydroxyde de sodium) au milieu d'époxydation, comme recommandé dans la demande de brevet WO 99/48882 de la demanderesse (dont le contenu est incorporé par référence dans la présente demande de brevet).

La réaction entre l'oléfine et le composé peroxydé peut s'effectuer en présence d'un sel tel que le chlorure de sodium, comme décrit dans la demande de brevet WO EP99/08703 de la demanderesse (dont le contenu est incorporé par référence dans la présente demande de brevet).

Il peut être avantageux d'introduire l'oléfine dans le réacteur, dans lequel la réaction d'époxydation a lieu, à l'état dilué dans un ou plusieurs alcanes. Par exemple, on peut introduire dans le réacteur d'époxydation un fluide contenant l'oléfine et également au moins 10 % (en particulier 20 %, par exemple au moins 30 %) en volume d'un ou plusieurs alcanes. Par exemple, dans le cas du propylène, celui-ci peut être mélangé avec au moins 10 % en volume de propane lorsqu'on introduit dans le réacteur le propylène non converti recyclé. Il peut également s'agir d'une source de propylène incomplètement épurée en propane.

### Exemple 1 (non conforme à l'invention)

Dans un réacteur double enveloppe en pyrex équipé d'un agitateur à pales et surmonté d'un réfrigérant refroidi à- 20 °C, on introduit 0.97 g de TS-1 et 38.4 g de méthanol (1.2 mol). La suspension est agitée à 750 rpm et la température est fixée à 25 °C. On introduit ensuite du propylène à un débit de 6 IN/h via un tube fritté. Après 30 min de balayage sous propylène (Pe), 7.4 g d'une solution de peroxyde d'hydrogène à 34.4 % poids (0.075 mole) sont ajoutés en 20 min.

La teneur en peroxyde d'hydrogène est déterminée par iodométrie après 90 min. La teneur en oxyde de propylène (PO) de la phase gazeuse est mesurée en ligne par chromatographie en phase vapeur. La phase liquide contenant l'oxyde de propylène et les sous-produits (méthoxypropanols "MeOPols" et propylène glycol "Diol") est analysée par chromatographie en phase vapeur en fin d'essai. Les résultats sont rassemblés dans le Tableau 1.

### Exemples 2 à 4 (conformes à l'invention)

On procède comme à l'exemple 1 sauf que le solvant est constitué d'un mélange de méthanol (MeOH) et d'acétonitrile (MeCN). Les résultats sont rassemblés dans le Tableau 1.

**Tableau 1**

| Ex. | MeOH/MeCN Rapport molaire | Taux de conversion d'H₂O₂ après 90 min (mol %) | Sélectivité en époxyde (mol %) | Teneur en sous-produits MeOPols+Diol (mol %) |
|---|---|---|---|---|
| 1 | 100/0 | 99.6 | 83.7 | 16.3 |
| 2 | 99.8/0.2 | 98.9 | 87.4 | 12.6 |
| 3 | 99/1 | 98.7 | 91.3 | 8.7 |
| 4 | 95/5 | 94.3 | 94.9 | 5.1 |

On n'a pas détecté la présence d'acétamide dans le milieu réactionnel en fin de réaction.

### Exemple 5 (conforme à l'invention)

On procède comme à l'exemple 1 sauf que le solvant est constitué d'un mélange de méthanol et de pivalonitrile au rapport de 99/1 mol/mol. Après 90 min de réaction, le taux de conversion du peroxyde est de 92.6 % mol. Les sélectivités sont respectivement de 92.2 mol % (oxyde de propylène) et 7.8 mol % (méthoxypropanols et propylène glycol).

On n'a pas détecté la présence d'acétamide dans le milieu réactionnel en fin de réaction.

### Exemple 6 (non conforme à l'invention)

Dans le réacteur de l'exemple 1, on introduit 1.20 g de TS-1, 38.4 g de méthanol (1.2 mol) et 11.5 g de chlorure d'allyle (0.15 mol). La suspension est agitée à 750 rpm et la température est fixée à 50 °C. On introduit ensuite en 20 min, 6.6 g d'une solution de peroxyde d'hydrogène à 38.6 % poids (0.075 mole).

La teneur en peroxyde d'hydrogène est déterminée par iodométrie après 30 min. La phase liquide contenant l'épichlorhydrine (EPI) et les sous-produits (chlorométhoxypropanols et chloropropanediol) est analysée par chromatographie en phase vapeur en fin d'essai.

Après 30 min de réaction, le taux de conversion du peroxyde est de 100 % mol. Les sélectivités sont respectivement de 98.4 mol % (épichlorhydrine) et 1.6 mol % (chlorométhoxypropanols et chloropropanediol).

### Exemple 7 (conforme à l'invention)

On procède comme à l'exemple 6 sauf que le solvant est constitué d'un mélange de méthanol et d'acétonitrile au rapport de 99/1 mol/mol. Après 30 min de réaction, le taux de conversion du peroxyde est de 100 % mol. Les sélectivités sont respectivement de 99.6 mol % (épichlorhydrine) et 0.4 mol % (chlorométhoxypropanols et chloropropanediol).

On n'a pas détecté la présence d'acétamide dans le milieu réactionnel en fin de réaction.

### Exemple 8 à 10 (conformes à l'invention)

Dans un réacteur continu du type boucle à bullosiphon tel que décrit dans la demande de brevet WO 9/48883, on introduit 4.5 g de billes de silice de 0.4-0.6 mm de diamètre contenant 1.5 g de TS-1. Le débit d'H₂O₂ mis en oeuvre à l'état de solution aqueuse à 39 % est de 0.174 mol/h. Une alimentation continue en diluant (= méthanol) est assurée pour maintenir un rapport CH₃OH/H₂O₂ = 16 mol/mol.

Suivant l'exemple (voir tableau 2), ce CH₃OH est ajouté pur ou additionné de 0.3 % ou 1 % mol d'acétonitrile.

La température des essais est maintenue à 55°C. Un débit important de Pe est mis en oeuvre (19.6 mol/mol d'H₂O₂, soit 75 IN/h). Ce gaz permet par bullosiphon de faire circuler le mélange réactionnel contenant le catalyseur en suspension et aussi de sortir en continu l'OP formé suivant la demande de brevet WO 99/48883.

On n'a pas détecté la présence d'acétamide dans le milieu réactionnel en fin de réaction.

**Tableau 2**

| Exemple | | 8 (ref) | 9 | 10 |
|---|---|---|---|---|
| % mol CH₃CN/CH₃OH | | 0 | 0.3 | 1 |
| Taux de conv H₂O₂ | après 6 h | 64.6 | 60.8 | 55.0 |
| | après 30 h | 47.8 | 45.5 | 41.6 |
| Sélectivité en OP* | après 6 h | 85 | 90 | 92 |
| | après 30 h | 88 | 92 | 93 |

| | | | | |
|---|---|---|---|---|
| * = % mol OP/Σ (OP + sous-produits) | | | | |

## Claims

1. Process for manufacturing an oxirane, in which an olefin is reacted, in a diluent chosen from water, alcohols and ketones, with a peroxide compound in the presence of a catalyst based on titanium silicalite of TS-1 type and in the presence of a nitrile.

2. Process according to Claim 1, in which the molar ratio of the amounts of nitrile and of diluent used is at least 0.001% and not more than 50%.

3. Process according to Claim 2, in which the molar ratio of the amounts of nitrile and of diluent used is at least 0.01% and not more than 10%.

4. Process according to any one of the preceding claims, in which the total amount of diluent and of nitrile used is at least 35% and not more than 99% by weight of the liquid phase containing the diluent, the nitrile, the dissolved olefin, the peroxide compound, a fraction of the oxirane formed and water.

5. Process according to any one of the preceding claims, in which the nitrile is chosen from linear or branched, saturated aliphatic nitriles and from aromatic nitriles, and the diluent is chosen from linear or branched, saturated aliphatic alcohols.

6. Process according to Claim 5, in which the nitrile contains from 2 to 7 carbon atoms and the alcohol contains from 1 to 6 carbon atoms.

7. Process according to Claim 6, in which the nitrile is acetonitrile and the diluent is methanol.

8. Process according to any one of the preceding claims, in which the olefin reacts with the peroxide compound in the presence of the catalyst, the diluent and the nitrile at a temperature of from 10°C to 100°C and at a pressure which may range from atmospheric pressure to 40 bar.

9. Process according to any one of the preceding claims, in which the oxirane is 1,2-epoxypropane, the olefin is propylene and the peroxide compound is hydrogen peroxide.

10. Process according to any one of Claims 1 to 8, in which the oxirane is 1,2-epoxy-3-chloropropane, the olefin is allyl chloride and the peroxide compound is hydrogen peroxide.

## Revendications

1. Procédé de fabrication d'un oxiranne selon lequel on fait réagir dans un diluant choisi parmi l'eau, les alcools et les cétones, une oléfine avec un composé peroxydé en présence d'un catalyseur à base de silicalite de titane de type TS-1 et en présence d'un nitrile.

2. Procédé selon la revendication 1, dans lequel le rapport molaire des quantités de nitrile et de diluant mises en oeuvre est d'au moins 0,001 et d'au plus 50 %.

3. Procédé selon la revendication 2, dans lequel le rapport molaire des quantités de nitrile et de diluant mises en oeuvre est d'au moins 0,01 et d'au plus 10 %.

4. Procédé selon l'une quelconque des revendications précédentes, dans lequel la quantité totale de diluant et de nitrile mise en oeuvre est d'au moins 35 % et d'au plus 99 % en poids de la phase liquide contenant le diluant, le nitrile, l'oléfine dissoute, le composé peroxydé, une fraction de l'oxiranne formé et de l'eau.

5. Procédé selon l'une quelconque des revendications précédentes, dans lequel le nitrile est choisi parmi les nitriles aliphatiques saturés, linéaires ou branchés et parmi les nitriles aromatiques, et le diluant est choisi parmi les alcools aliphatiques saturés, linéaires ou branchés.

6. Procédé selon la revendication 5, dans lequel le nitrile contient de 2 à 7 atomes de carbone, et l'alcool contient de 1 à 6 atomes de carbone.

7. Procédé selon la revendication 6, dans lequel le nitrile est l'acétonitrile et le diluant est le méthanol.

8. Procédé selon l'une quelconque des revendications précédentes, dans lequel l'oléfine réagit avec le composé peroxydé en présence du catalyseur, du diluant et du nitrile à une température de 10 °C à 100 °C, et à une pression qui peut varier de la pression atmosphérique à 40 bar.

9. Procédé selon l'une quelconque des revendications précédentes, dans lequel l'oxiranne est le 1,2-époxypropane, l'oléfine est le propylène et le composé peroxydé est le peroxyde d'hydrogène.

10. Procédé selon l'une quelconque des revendications 1 à 8, dans lequel l'oxiranne est le 1,2-époxy-3-chloropropane, l'oléfine est le chlorure d'allyle et le composé peroxydé est le peroxyde d'hydrogène.

## Patentansprüche

1. Verfahren zur Herstellung eines Oxirans, worin in einem unter Wasser, Alkoholen und Ketonen ausgewählten Verdünnungsmittel ein Olefin mit einer Peroxidverbindung in Anwesenheit eines Katalysators aufTitansilicalitbasis vom Typ TS-1 und in Anwesenheit eines Nitrils zur Reaktion gebracht wird.

2. Verfahren nach Anspruch 1, worin das Molverhältnis der eingesetzten Mengen an Nitril und Verdünnungsmittel wenigstens 0,001 und höchstens 50% beträgt.

3. Verfahren nach Anspruch 2, worin das Molverhältnis der eingesetzten Mengen an Nitril und Verdünnungsmittel wenigstens 0,01 und höchstens 10% beträgt.

4. Verfahren nach einem der vorstehenden Ansprüche, worin die Gesamtmenge an eingesetztem Verdünnungsmittel und Nitril wengistens 35% und höchstens 99 Gew.-% der flüssigen Phase beträgt, die das Verdünnungsmittel, das Nitril, das gelöste Olefin, die Peroxidverbindung, einen Teil des gebildeten Oxirans und Wasser enthält.

5. Verfahren nach einem der vorstehenden Ansprüche, worin das Nitril unter gesättigten, geraden oder verzweigten aliphatischen Nitrilen und unter aromatischen Nitrilen ausgewählt wird und das Verdünnungsmittel unter gesättigten, geradenoder verzweigten aliphatischen Alkoholen ausgewählt wird.

6. Verfahren nach Anspruch 5, worin das Nitril 2 bis 7 Kohlenstoffatome enthält und der Alkohol 1 bis 6 Kohlenstoffatome enthält.

7. Verfahren nach Anspruch 6, worin das Nitril Acetonitril ist und das Verdünnungsmittel Methanol ist.

8. Verfahren nach einem der vorstehenden Ansprüche, worin das Olefin mit der Peroxidverbindung in Anwesenheit von Katalysator, Verdünnungsmittel und Nitril bei einer Temperatur von 10°C bis 100°C und bei einem Druck, der von Atmosphärendruck bis zu 40 bar variieren kann, reagiert.

9. Verfahren nach einem der vorstehenden Ansprüche, worin das Oxiran 1,2-Epoxypropan ist, das Olefin Propylen ist und die Peroxidverbindung Wasserstoffperoxid ist.

10. Verfahren nach einem der Ansprüche 1 bis 8, worin das Oxiran 1,2-Epoxy-3-chlorpropan ist, das Olefin Allylchlorid ist und die Peroxidverbindung Wasserstoffperoxid ist.
